# EUROPEAN PATENT SPECIFICATION

(11) **EP 2 125 038 B1**
(45) Date of publication and mention of the grant of the patent: **02.05.2012**
(21) Application number: 07848610.7
(22) Date of filing: 19.12.2007
(51) Int. Cl.: A61K 51/04, C07D 211/62, A61K 101/02

(54) **RADIOACTIVE PET AGENTS FOR IMAGING CCR5 IN VIVO**
RADIOAKTIVE MITTEL FÜR DIE IN VIVO PET-BILDGEBUNG VON CCR5
AGENTS RADIOACTIFS POUR L'IMAGERIE PET IN VIVO DES RECEPTEURS CCR5

(30) Priority: 21.12.2006 GB 0625523
(43) Date of publication of application: 02.12.2009
(73) Proprietor: GE Healthcare Limited, Little Chalfont Buckinghamshire HP7 9NA (GB); Hammersmith Imanet Limited, London W12 0NN (GB)
(72) Inventor: STOREY, Tony, Amersham Buckinghamshire HP7 9LL (GB); DAVIS, Julie, Amersham Buckinghamshire HP7 9LL (GB); ARSTAD, Erik, London Greater London W12 0NN (GB); GUILBERT, Benedicte, Amersham Buckinghamshire HP7 9LL (GB); GAETA, Alessandra, Amersham Buckinghamshire HP7 9LL (GB)
(74) Representative: Canning, Lewis R.
(86) International application number: PCT/GB2007/004880
(87) International publication number: WO 2008/075040

(56) References cited:
- EP-A- 1 236 726
- WO-A-00/06146
- WO-A-00/66558
- WO-A-01/25200
- WO-A-01/66525
- WO-A-02/10146
- WO-A-03/042177
- WO-A-03/042178
- WO-A-2006/102395
- SIGNORE A ET AL: "Radiolabelled lymphokines and growth factors for in vivo imaging of inflammation, infection and cancer" TRENDS IN IMMUNOLOGY, ELSEVIER, RAHWAY, NJ, US, vol. 24, no. 7, 1 July 2003 (2003-07-01), pages 395-402, XP004437496 ISSN: 1471-4906
- IMAMURA S ET AL: "CCR5 antagonists as anti-HIV-1 agents. Part 3: Synthesis and biological evaluation of piperidine-4-carboxamide derivatives" BIOORGANIC & MEDICINAL CHEMISTRY, ELSEVIER SCIENCE LTD, GB, vol. 13, no. 2, 17 January 2005 (2005-01-17), pages 397-416, XP004681524 ISSN: 0968-0896 cited in the application
- CUMMING J G ET AL: "Modulators of the human CCR5 receptor. Part 3: SAR of substituted 1-[3-(4-methanesulfonylphenyl)-3-phenylpro pyl]-piperidinyl phenylacetamides" BIOORGANIC & MEDICINAL CHEMISTRY LETTERS, PERGAMON, ELSEVIER SCIENCE, GB, vol. 16, no. 13, 1 July 2006 (2006-07-01), pages 3533-3536, XP025107407 ISSN: 0960-894X [retrieved on 2006-07-01] cited in the application
- RIBEIRO S ET AL: "The clinical potential of chemokine receptor antagonists" PHARMACOLOGY AND THERAPEUTICS, ELSEVIER, GB, vol. 107, no. 1, 1 July 2005 (2005-07-01), pages 44-58, XP004930038 ISSN: 0163-7258 cited in the application
- LU ET AL: "CCR5 receptor antagonists: Discovery and SAR of novel 4-hydroxypiperidine derivatives" BIOORGANIC & MEDICINAL CHEMISTRY LETTERS, PERGAMON, ELSEVIER SCIENCE, GB, vol. 17, no. 7, 12 March 2007 (2007-03-12), pages 1883-1887, XP005922506 ISSN: 0960-894X cited in the application
- TAGAT J R ET AL: "Piperazine-Based CCR5 Antagonists as HIV-1 Inhibitors. IV. Discovery of 1-[(4,6-Dimethyl-5-pyrimidinyl) carbonyl]-4-[4-{2-methoxy-1(R)-4-( trifluoromethyl)-phenyl}ethyl-3(S) -methyl-1-piperazinyl]-4-methylpip eridine (Sch-417690/Sch-D), a Potent, Highly Selective, and Orally Bioavailable CCR5 Antago" JOURNAL OF MEDICINAL CHEMISTRY 20040506 US, vol. 47, no. 10, 6 May 2004 (2004-05-06), pages 2405-2408, XP002543503 ISSN: 0022-2623 cited in the application
- TAGAT J R ET AL: "Piperazine-based CCR5 antagonists as HIV-1 inhibitors. II. Discovery of 1-[(2,4-dimethyl-3-pyridinyl)carbonyl]-4me thyl-4-[3(S)-methyl-4-[1 (S)-[4- (trifluoromethyl) phenyl]ethyl]-1-piperazinyl]piperidine N1-oxide (Sch-350634), an orally bioavailable, potent CCR5 antagonist" JOURNAL OF MEDICINAL CHEMISTRY 20011011 US, vol. 44, no. 21, 11 October 2001 (2001-10-11), pages 3343-3346, XP002543504 ISSN: 0022-2623 cited in the application
- WOOD ANTHONY ET AL: "The discovery of the CCR5 receptor antagonist, UK-427,857, a new agent for the treatment of HIV infection and AIDS." PROGRESS IN MEDICINAL CHEMISTRY 2005, vol. 43, 2005, pages 239-271, XP008111034 ISSN: 0079-6468 cited in the application
- NISHIZAWA ET AL: "Spirodiketopiperazine-based CCR5 antagonists: Lead optimization from biologically active metabolite" BIOORGANIC & MEDICINAL CHEMISTRY LETTERS, PERGAMON, ELSEVIER SCIENCE, GB, vol. 17, no. 3, 2007, pages 727-731, XP005835927 ISSN: 0960-894X cited in the application
- STRIZKI J M ET AL: "Discovery and characterization of vicriviroc (SCH 417690), a CCR5 antagonist with potent activity against human immunodeficiency virus type 1" ANTIMICROBIAL AGENTS AND CHEMOTHERAPY 200512 US, vol. 49, no. 12, December 2005 (2005-12), pages 4911-4919, XP002543506 ISSN: 0066-4804
- BOLTON R: "Radiohalogen incorporation into organic systems" JOURNAL OF LABELLED COMPOUNDS AND RADIOPHARMACEUTICALS, JOHN WILEY, CHICHESTER, GB, vol. 45, no. 6, 1 May 2002 (2002-05-01), pages 485-528, XP002484962 ISSN: 0362-4803

## Description

### Technical Field of the Invention

The present invention relates to the field of medical imaging, and in particular to *in vivo* imaging of disease states associated with the upregulation of a particular class of chemokine receptor (CCR). Compounds and methods are provided that are useful for imaging such disease states.

### Description of Related Art

The chemokine system regulates the trafficking of immune cells to tissues and thus plays a central role in inflammation. The system is also involved in many other biological processes such as growth regulation, haematopoiesis and angiogenesis. In addition, chemokines are thought to play a central role in the central nervous system. Chemokines (chemotactic cytokines) are small secreted molecules characterised by 4 conserved cysteine residues forming two essential disulphide bonds (Cys1-Cys3; Cys2-Cys4). They can be briefly classified, based on the relative position of the two cysteine residues, as CC and CXC, which represent the two major classes. Chemokines act as chemical mediators, released either by invading immune cells or by resident cells locally at the site of inflammation.

Chemokines induce their biological effects through interaction with chemokine receptors (CCR). CCR are integral membrane proteins, formed of seven transmembrane α-helix domains linked by intracellular and extracellular loops, an extracellular N-terminus and a cytosolic C-terminus. They all share a common fold of three stranded antiparallel β-sheets covered on one face by a C-terminus α-helix and preceded by a disordered N-terminus. The dimerisation/oligomerisation process, essential for their functional properties, involves the N-terminus.

Expression of chemokine receptors (CCR) has been found to be perturbed in certain disease states where inflammation plays a role. For example, neuroinflammatory diseases such as multiple sclerosis (MS) [Rottman et al 2000 Eur. J. Immunol. 30 p2372], Alzheimer's disease (AD) and Parkinson's disease (PD), [Xia & Hyman 1999 J. Neurovirology 5 p32] and also other pathological inflammatory conditions such as atherosclerosis [Greaves & Channon 2002 Trends Immunol. 23(11) p535], chronic obstructive pulmonary disorder (COPD), rheumatoid arthritis, osteoarthritis, allergic disease, HIV/AIDS, asthma and cancer.

One chemokine receptor that is particularly important in certain disease states is CCR5. It has been the subject of considerable therapeutic development as it is the chemokine receptor which the human immunodeficiency virus (HIV) uses to gain entry into macrophages and CCR5 expression is upregulated in chronic HIV infection. CCR5 has also received attention due to its involvement in the pathophysiology of various neuroinflammatory conditions such as MS, Alzheimer's disease and PD.

Chemokine receptor ligands have been reviewed by Gao and Metz [Chem.Rev., 103, 3733-52 (2003)], and Ribeiro and Horuk [Pharmacol.Ther., 107, 44-58 (2005)].

Targeting cytokine and chemokine receptors for nuclear medical imaging has been described as a challenge [Signore et al, Eur.J.Nucl.Med.Mol.Imaging, 30(1), 149-165 (2003)]. Signore *et al* reported that the main approach known to target chemokine receptors was radiolabelled interleukin-8 (IL-8).

WO 02/36581 teaches radiopharmaceuticals that bind to the CCR1 receptor and that are able to pass through the blood-brain barrier (BBB). These radiopharmaceuticals are taught as useful in diagnosing Alzheimer's disease.

WO 2006/102395 teaches targeting of imaging moieties (referred to therein as "imaging agents") to atherosclerotic plaques. The ligand RANTES, which binds to the CCR5 receptor, is taught as one of a number of targeting moieties suitable for the delivery of an imaging moiety to atherosclerotic lesions when linked thereto. The imaging moieties taught include those suitable for a range of *in vivo* imaging modalities, e.g. single photon emission tomography (SPECT), magnetic resonance imaging (MRI) and positron emission tomography (PET).

Signore et al ("Radiolabelled lymphokines and growth factors for in vivo imaging of inflammation, infection and cancer": TRENDS in Immunology 2003, vol.24, No. 7, pp. 395-402) makes reference to the ligand RANTES labelled with ¹²⁵I for inflammation imaging. WO 01/25200 and Imamura et al (Bioorg. & Med. Chem. Lett. 13 (2005), pp. 397-416) disclose non-radiolabelled compounds being CCR5 ligands.

The ability to image conditions where CCR5 is specifically implicated, especially neuroinflammation, may represent an important tool for early diagnosis of different acute and chronic pathological conditions and to support therapeutic approaches and strategies. There is therefore a need for imaging agents which image CCR5, and in particular those that can cross the BBB.

### Summary of the Invention.

The present invention relates to *in vivo* imaging and in particular to novel imaging agents suitable for use in *in vivo* imaging of the chemokine receptor 5 (CCR5). The invention also provides a method for the preparation of the imaging agents of the invention as well as pharmaceutical compositions comprising them. For the facile preparation of the pharmaceutical compounds, kits are provided. In addition, the invention provides methods for the use of the imaging agents and pharmaceutical compositions of the invention.

### Detailed Description of the Invention.

In a first aspect, the present invention provides an imaging agent as defined in claim 1 which comprises a synthetic compound having affinity for chemokine receptor 5 (CCR5) and having a molecular weight of 3000 Daltons or less.

A compound having "affinity for CCR5" is defined in the present invention as that which inhibits binding of MIP-1β CCR5-expressing CHO cells with IC₅₀ values of between 0.1nM to 10nM, where MIP-1β is Macrophage Inflammatory Protein 1β (ligand of CCR5) [Samson et al., J. Biol. Chem., 272, 24934-41 (1997)]. See also Example 4. The CCR5 compounds of the present invention are also preferably selective for CCR5 over other chemokine receptors (such as CCR1 or CCR3). Such selective inhibitors suitably exhibit a greater potency for CCR5 over CCR1, defined by Ki, of a factor of at least 50, preferably at least 100, most preferably at least 500.

The synthetic compound is a non-peptide. By the term "non-peptide" is meant a compound which does not comprise any peptide bonds, i.e. an amide bond between two amino acid residues. The synthetic compound having affinity for chemokine receptor 5 (CCR5) preferably has a molecular weight of 1000 Daltons or less, and most preferably 600 Daltons or less.

The term "labelled with" means that either a functional group comprises the imaging moiety, or the imaging moiety is attached as an additional species. When a functional group comprises the imaging moiety, this means that the 'imaging moiety' forms part of the chemical structure, and is a radioactive isotope present at a level significantly above the natural abundance level of said isotope. Such elevated or enriched levels of isotope are suitably at least 5 times, preferably at least 10 times, most preferably at least 20 times; and ideally either at least 50 times the natural abundance level of the isotope in question, or present at a level where the level of enrichment of the isotope in question is 90 to 100%. Reference examples of such functional groups include CH₃ groups with elevated levels of ¹¹C, and fluoroalkyl groups with elevated levels of ¹⁸F, such that the imaging moiety is the isotopically labelled ¹¹C or ¹⁸F atom within the chemical structure. The radioisotopes ³H and ¹⁴C are not suitable imaging moieties.

The imaging moiety of the present invention is a positron-emitting radioactive non-metal, which is suitable for positron emission tomography (PET). Positron emitters include: ¹¹C, ¹³N, ¹⁷F. ¹⁸F, ⁷⁵Br, ⁷⁶Br or ¹²⁴I. .

The positron emitter used as imaging moiety in the present invention is ¹⁸F. The use of such a PET imaging moiety has certain technical advantages, including:
(i) the development of PET/CT cameras allowing easy co-registration of functional (PET) and anatomical (CT) images for improved diagnostic information;
(ii) the facility to quantify PET images to allow accurate assessment for staging and therapy monitoring;
(iii) increased sensitivity to allow visualisation of smaller target tissues.

The imaging agent of the present invention comprises a synthetic compound of formula II: 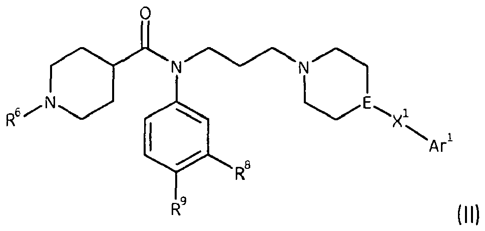 wherein:
R⁶ is [¹⁸F]fluoroacyl;
R⁸-R⁹ are independently selected from H, C₁₋₃ alkyl, OH or halogen;
E is N or CH;
when E is N, X¹ is -CH₂-and when E is CH, X¹ is -CH₂- or -O-;
Ar¹ is a phenyl ring substituted with 0 to 3 R⁷groups;
each R⁷ is independently chosen from C₁₋₃ alkyl, OH, halogen, NO₂, NH₂, CO₂H, C₁-₆alkoxy, C₁₋₆ amino, C₁₋₆ amido, -O(CH₂CH₂OₓX² or -NH(CH₂CH₂O)ₓX² where x is an integer of value 0 to 4, and X² is H or CH₃;
wherein said compound has affinity for chemokine receptor 5 (CCR5) and a molecular weight of 3000 Daltons or less.

In Formula II, R⁸-R⁹ are preferably selected from H, CH₃, OH, Cl, F and I; and Ar¹ is a phenyl ring. Preferably, the Ar¹ ring is unsubstituted or substituted with one R⁷ group. When present, R⁷ is preferably chosen from: -OH, -NHCH₃, F, -O(CH₂CH₂O)ₓX² or - NH(CH₂CH₂O)ₓX². X² is preferably H.

The preferred compound of Formula II is:

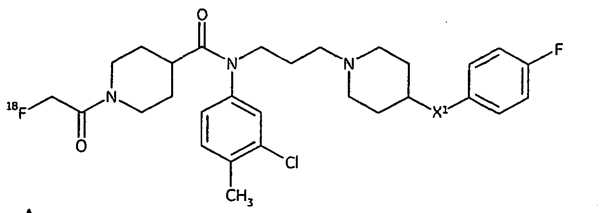

Other reference compounds are: 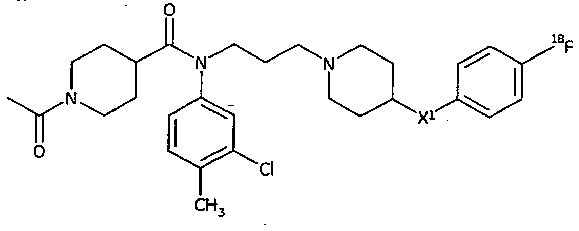 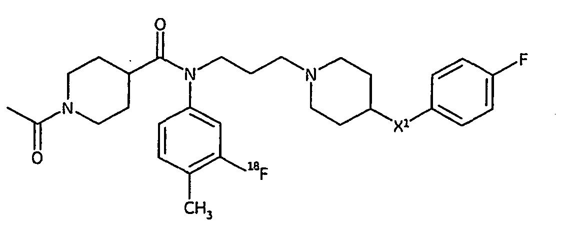 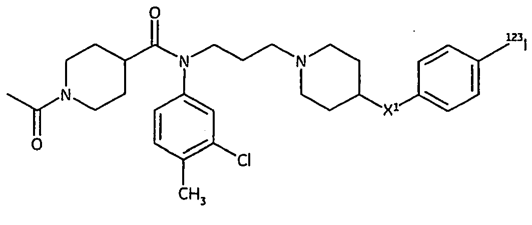 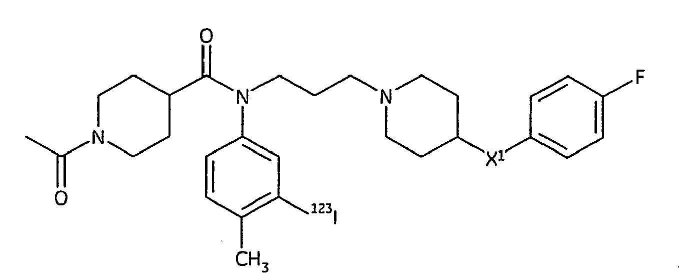 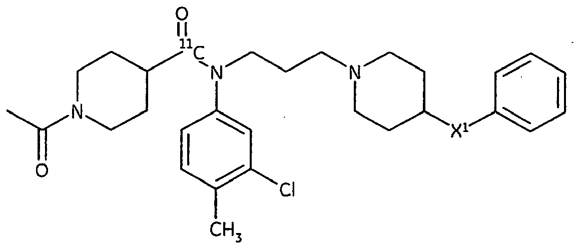 wherein X¹ is as defined above for Formula II.

Synthetic compounds having affinity for chemokine receptor 5 (CCR5) can be obtained as follows:
Formula I - WO 00/06146, Shiraishi et al [J. Med. Chem. 43 pp2049-63 (2000)].
Formula II Piperidine-4-carboxamide derivatives, Imamura et al, [Bioorg. Med. Chem. 13 p. 397-416 (2005), and J. Med. Chem. 49 pp2784-93 (2006)].
Formula III - diphenylpropylpiperidine derivatives, Cumming et al [Bioorg. Med. Chem. Lett, 16 p3533 - 3536 (2006)], and Shou-Fu Lu et al. Bioorg. Med. Chem.Lett, 2007,17, 1883-1887.
Formula IV - piperazine-based derivatives, Tagat et al [J.Med.Chem., 47, 2405-8 (2004)].; and Tagat et al [J. Med. Chem 44, 3343-6 (2001)]
Formula V - Wood and Armour [Prog.Med.Chem., 43, 239-271 (2005)]
Formula VI - Mitsuya et al [J. Med. Chem. 49 pp4140-52 (2006), and Bioorg. Med. Chem. Lett. 17 pp727-31 (2007)]

Only formula II referred to above is related to the present invention.

The imaging agents of me first aspect are suitably prepared by reaction with a precursor, as described in the second aspect below.

In a second aspect, the present invention provides a method for the preparation of the imaging agent of the first aspect and referred to in present claim 1 which comprises reaction of:
(i) a non-radioactive precursor; and,
(ii) a suitable source of the imaging moiety of the first aspect,
wherein said precursor is a derivative of the synthetic compound of present claim 1, and said derivative comprises a substituent Y¹ which is capable of reaction with said suitable source of the imaging moiety to give the desired imaging agent.

The "precursor" suitably comprises a non-radioactive derivative of the synthetic compound, which is designed so that chemical reaction with a convenient chemical form of the desired non-metallic radioisotope can be conducted in the minimum number of steps (ideally a single step), and without the need for significant purification (ideally no further purification) to give the desired radioactive product. Such precursors are synthetic and can conveniently be obtained in good chemical purity. The "precursor" may optionally comprise a protecting group (P^{GP}) for certain functional groups of the synthetic CCR5 compound. Suitable precursors are described by Bolton, J.Lab.Comp.Radiopharm., 45, 485-528 (2002).

By the term "protecting group" (P^{GP}) is meant a group which inhibits or suppresses undesirable chemical reactions, but which is designed to be sufficiently reactive that it may be cleaved from the functional group in question under mild enough conditions that do not modify the rest of the molecule. After deprotection the desired product is obtained. Protecting groups are well known to those skilled in the art and are suitably chosen from, for amine groups: Boc (where Boc is *tert-*butyloxycarbonyl), Fmoc (where Fmoc is fluorenylmethoxycarbonyl), trifluoroacetyl, allyloxycarbonyl, Dde [i.e. 1-(4,4-dimethyl-2,6-dioxocyclohexylidene)ethyl] or Npys (i.e. 3-nitro-2-pyridine sulfenyl); and for carboxyl groups: methyl ester, tert-butyl ester or benzyl ester. For hydroxyl groups, suitable protecting groups are: methyl, ethyl or tert-butyl; alkoxymethyl or alkoxyethyl; benzyl; acetyl; benzoyl; trityl (Trt) or trialkylsilyl such as tert-butyldimethylsilyl. For thiol groups, suitable protecting groups are: trityl and 4-methoxybenzyl. The use of further protecting groups are described in 'Protective Groups in Organic Synthesis', Theorodora W. Greene and Peter G. M. Wuts, (Third Edition, John Wiley & Sons, 1999).

Reference precursors are those wherein Y¹ comprises a derivative which either undergoes direct electrophilic or nucleophilic halogenation; undergoes facile alkylation with a labelled alkylating agent chosen from an alkyl or fluoroalkyl halide, tosylate, triflate (i.e. trifluoromethanesulphonate), mesylate, maleimide or a labelled N-haloacetyl moiety; alkylates thiol moieties to form thioether linkages; or undergoes condensation with a labelled active ester, aldehyde or ketone. Examples of the first category are:
(a) organometallic derivatives such as a trialkylstannane (e.g. trimethylstannyl or tributylstannyl), or a trialkylsilane (e.g. trimethylsilyl);
(b) a non-radioactive alkyl iodide or alkyl bromide for halogen exchange and alkyl tosylate, mesylate or triflate for nucleophilic halogenation;
(c) aromatic rings activated towards electrophilic halogenation (e.g. phenols) and aromatic rings activated towards nucleophilic halogenation (e.g. aryl iodonium, aryl diazonium, aryl trialkylammonium salts or nitroaryl derivatives).

Reference derivatives which undergo facile alkylation are alcohols, phenols, amine or thiol groups, especially thiols and sterically-unhindered primary or secondary amines.

Reference derivatives which alkylate thiol-containing radioisotope reactants are maleimide derivatives or N-haloacetyl groups. Particular reference examples of the latter are N-chloroacetyl and N-bromoacetyl derivatives.

Other reference derivatives which undergo condensation with a labelled active ester moiety are amines, especially sterically-unhindered primary or secondary amines.

Particular reference derivatives which undergo condensation with a labelled aldehyde or ketone are aminooxy and hydrazides groups, especially aminooxy derivatives.

The "precursor" may optionally be supplied covalently attached to a solid support matrix. In that way, the desired imaging agent product forms in solution, whereas starting materials and impurities remain bound to the solid phase. Precursors for solid phase electrophilic fluorination with ¹⁸F-fluoride are described in WO 03/002489. Precursors for solid phase nucleophilic fluorination with ¹⁸F-fluoride are described in WO 03/002157. The solid support-bound precursor may therefore be provided as a kit cartridge which can be plugged into a suitably adapted automated synthesizer. The cartridge may contain, apart from the solid support- bound precursor, a column to remove unwanted fluoride ion, and an appropriate vessel connected so as to allow the reaction mixture to be evaporated and allow the product to be formulated as required. The reagents and solvents and other consumables required for the synthesis may also be included together with a compact disc carrying the software which allows the synthesiser to be operated in a way so as to meet the customer requirements for radioactive concentration, volumes, time of delivery etc. Conveniently, all components of the kit are disposable to minimise the possibility of contamination between runs and will be sterile and quality assured.

Methods of introducing radioactive halogens (including ¹²³I and ¹⁸F) are described by Bolton [J.Lab.Comp.Radiopharm., 45, 485-528 (2002)]. Reference examples of suitable precursor aryl groups to which radioactive halogens, especially iodine can be attached are given below:

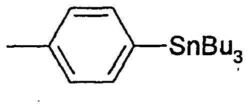

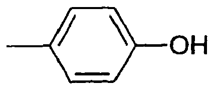

Both contain substituents which permit facile radioiodine substitution onto the aromatic ring. Alternative substituents containing radioactive iodine can be synthesised by direct iodination via radiohalogen exchange, e.g.

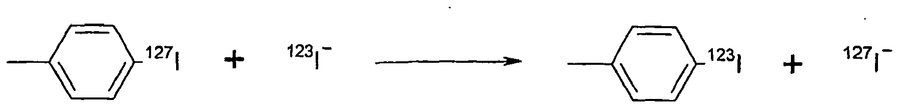

In reference embodiments the radiofluorine atom may form part of a fluoroalkyl or fluoroalkoxy group, since alkyl fluorides are resistant to *in vivo* metabolism. For radioactive isotopes of fluorine (e.g. ¹¹³F), the radiohalogenation in reference embodiments may be carried out via direct labelling using the reaction of ¹⁸F-fluoride with a suitable precursor having a good leaving group, such as an alkyl bromide, alkyl mesylate or alkyl tosylate. In other reference embodiments, the radiofluorine atom may be attached *via* a direct covalent bond to an aromatic ring such as a benzene ring. For such aryl systems, the precursor suitably comprises an activated nitroaryl ring, an aryl diazonium salt, or an aryl trialkylammonium salt. Direct radiofluorination can, however, be detrimental to sensitive functional groups since these nucleophilic reactions are carried out with anhydrous [¹⁸F]fluoride ion in polar aprotic solvents under strong basic conditions.

When the synthetic compound has alkali-sensitive functional groups, or other functionality unsuitable for direct radiohalogenation, an indirect radiohalogenation method is preferred. Thus, as the imaging moiety comprises a radioactive ¹⁸F, Y¹ preferably comprises a functional group that will react selectively with a radiolabelled synthon and thus upon conjugation gives the desired imaging agent product. By the term "radiolabelled synthon" is meant a small, synthetic organic molecule which is:
(i) already radiolabelled such that the radiolabel is bound to the synthon in a stable manner;
(ii) comprises a functional group designed to react selectively and specifically with a corresponding functional group which is part of the desired compound to be radiolabelled. This approach gives better opportunities to generate imaging agents with improved *in vivo* stability of the radiolabel relative to direct radiolabelling approaches.
A synthon approach also allows greater flexibility in the conditions used for the introduction of the imaging moiety.

In Reference embodiments, 18F-can be introduced by N-alkylation of amine precursors with alkylating agent synthons such as ¹⁸F(CH₂)₃OMs (where Ms is mesylate) to give N-(CH₂)₃¹⁸F, O-alkylation of hydroxyl groups with ¹⁸F(CH₂)₃OMs, ¹⁸F(CH₂)₃OTs or ¹⁸F(CH₂)₃Br or S-alkylation of thiol groups with ¹⁸F(CH₂)₃OMs or ¹⁸F(CH₂)₃Br. In other reference embodiments, ¹⁸F can be introduced by alkylation of N-haloacetyl groups with a ¹⁸F(CH₂)₃OH reactant, to give -NH(CO)CH₂O(CH₂)₃¹⁸F derivatives or with a ¹⁸F(CH₂)₃SH reactant, to give - NH(CO)CH₂S(CH₂)₃⁸F derivatives. In other reference embodiments, ¹⁸F can be introduced by reaction of maleimide-containing precursors with ¹⁸F(CH₂)₃SH. For aryl systems in reference examples, ¹⁸F-fluoride nucleophilic displacement from an aryl diazonium salt, an aryl nitro compound or an aryl quaternary ammonium salt are suitable routes to aryl-¹⁸F labelled synthons useful for conjugation to precursors of a reference imaging agent.

Reference Precursors wherein V¹ comprises a primary amine group can also be labelled with ¹⁸F by reductive amination using 18F-C₆H₄-CHO as taught by Kahn et al [J.Lab.Comp.Radiopharm. 45,1045-1053 (2002)] and Borch et al [J. Am. Chem. Soc. 93, 2897 (1971)]. This approach can also usefully be applied to aryl primary amines, such as compounds comprising phenyl-NH₂ or phenyl-CH₂NH₂ groups.

An especially preferred method for base-sensitive precursors is when reference Y¹ comprises an aminooxy group of formula -NH(C=O)CH₂-O-NH₂ which is condensed with ¹⁸F-C₆H₄-CHO under acidic conditions (e.g. pH 2 to 4). Further details of synthetic routes to ¹⁸F-labelled derivatives are described by Bolton, J.Lab.Comp.Radiopharm., 45, 485-528 (2002).

The precursor is preferably in sterile, apyrogenic form. Methods for maintaining sterility are described in the third aspect below.

Examples of precursors suitable for the generation of reference imaging agents are those where Y¹ comprises an amine group which is condensed with the synthon N-succinimidyl 4-[¹²³I]iodobenzoate at pH 7.5-8.5 to give amide bond linked products.

In a third aspect, the present invention provides a pharmaceutical composition which comprises the imaging agent of the first aspect together with a biocompatible carrier, in a form suitable for mammalian administration.

The "biocompatible carrier" is a fluid, especially a liquid, in which the imaging agent can be suspended or dissolved, such that the composition is physiologically tolerable, i.e. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is isotonic); an aqueous solution of one or more tonicity-adjusting substances (e.g. salts of plasma cations with biocompatible counterions), sugars (e.g. glucose or sucrose), sugar alcohols (e.g. sorbitol or mannitol), glycols (e.g. glycerol), or other non-ionic polyol materials (e.g. polyethyleneglycols, propylene glycols and the like). Preferably the biocompatible carrier is pyrogen-free water for injection or isotonic saline.

Such radioactive pharmaceutical compositions (i.e. radiopharmaceutical compositions) are suitably supplied in either a container which is provided with a seal which is suitable for single or multiple puncturing with a hypodermic needle (e.g. a crimped-on septum seal closure) whilst maintaining sterile integrity. Such containers may contain single or multiple patient doses. Preferred multiple dose containers comprise a single bulk vial (e.g. of 10 to 30 cm³ volume) which contains multiple patient doses, whereby single patient doses can thus be withdrawn into clinical grade syringes at various time intervals during the viable lifetime of the preparation to suit the clinical situation. Prefilled syringes are designed to contain a single human dose, or "unit dose" and are therefore preferably a disposable or other syringe suitable for clinical use. The prefilled syringe may optionally be provided with a syringe shield to protect the operator from radioactive dose. Suitable such radiopharmaceutical syringe shields are known in the art and preferably comprise either lead or tungsten.

The radiopharmaceutical compositions may be prepared from kits, as is described in the fourth aspect below. Alternatively, the radiopharmaceuticals may be prepared under aseptic manufacture conditions to give the desired sterile product. The radiopharmaceuticals may also be prepared under non-sterile conditions, followed by terminal sterilisation using e.g. gamma-irradiation, autoclaving, dry heat or chemical treatment (e.g. with ethylene oxide).

In a fourth aspect, the present invention provides a kit for the preparation of the pharmaceutical composition of the third aspect, which kit comprises the precursor of the second aspect Such kits comprise the "precursor" of the second aspect, preferably in sterile non-pyrogenic form, so that reaction with a sterile source of the radioisotopic imaging moiety gives the desired radiopharmaceutical with the minimum number of manipulations. Such considerations are particularly important when the radioisotope has a relatively short half-life, and for ease of handling and hence reduced radiation dose for the radiopharmacist. Hence, the reaction medium for reconstitution of such kits is preferably a "biocompatible carrier" as defined above, and is most preferably aqueous.

Suitable kit containers comprise a sealed container which permits maintenance of sterile integrity and/or radioactive safety, plus optionally an inert headspace gas (e.g. nitrogen or argon), whilst permitting addition and withdrawal of solutions by syringe. A preferred such container is a septum-sealed vial, wherein the gas-tight closure is crimped on with an overseal (typically of aluminium). Such containers have the additional advantage that the closure can withstand vacuum if desired e.g. to change the headspace gas or degas solutions.

The non-radioactive kits may optionally further comprise additional components such as a radioprotectant, antimicrobial preservative, pH-adjusting agent or filler. By the term "radioprotectant" is meant a compound which inhibits degradation reactions, such as redox processes, by trapping highly-reactive free radicals, such as oxygen-containing free radicals arising from the radiolysis of water. The radioprotectants of the present invention are suitably chosen from: ascorbic acid, para-aminobenzoic acid (i.e. 4-aminobenzoic acid), gentisic acid (i.e. 2,5-dihydroxybenzoic acid) and salts thereof with a biocompatible cation. By the term "biocompatible cation" is meant a positively charged counterion which forms a salt with an ionised, negatively charged group, where said positively charged counterion is also non-toxic and hence suitable for administration to the mammalian body, especially the human body. Examples of suitable biocompatible cations include: the alkali metals sodium or potassium; the alkaline earth metals calcium and magnesium; and the ammonium ion. Preferred biocompatible cations are sodium and potassium, most preferably sodium.

By-the-term "antimicrobial-pr-eservative" is meant an agentwhich inhibits the growth of potentially harmful micro-organisms such as bacteria, yeasts or moulds. The antimicrobial preservative may also exhibit some bactericidal properties, depending on the dose. The main role of the antimicrobial preservative(s) of the present invention is to inhibit the growth of any such micro-organism in the radiopharmaceutical composition post-reconstitution, i.e. in the radioactive diagnostic product itself. The antimicrobial-preservative may, however, also optionally be used to inhibit the growth of potentially harmful micro-organisms in one or more components of the non-radioactive kit of the present invention prior to reconstitution. Suitable antimicrobial preservative(s) include: the parabens, i.e. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds useful to ensure that the pH of the reconstituted kit is within acceptable limits (approximately pH 4.0 to 10.5) for human or mammalian administration. Suitable such pH-adjusting agents include pharmaceutically acceptable buffers, such as tricine, phosphate or TRIS [i.e. *tris*(hydroxymethyl)aminomethaneJ. and pharmaceutically acceptable bases such as sodium carbonate, sodium bicarbonate or mixtures thereof. When the conjugate is employed in acid salt form, the pH adjusting agent may optionally be provided in a separate vial or container, so that the user of the kit can adjust the pH as part of a multi-step procedure.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose.

Preferred aspects of the "precursor" when employed in the kit are as described for the second aspect above. The precursors for use in the kit may be employed under aseptic manufacture conditions to give the desired sterile, non-pyrogenic material. The precursors may also be employed under non-sterile conditions, followed by terminal sterilisation using e.g. gamma-irradiation, autoclaving, dry heat or chemical treatment (e.g. with ethylene oxide). Preferably, the precursors are employed in sterile, non-pyrogenic form. Most preferably the sterile, non-pyrogenic precursors are employed in the sealed container as described above.

In a fifth aspect, the present invention provides a pharmaceutical composition for use in a method for the *in vivo* diagnosis or imaging in a subject of a CCR5 condition, comprising administration of the pharmaceutical composition of the third aspect. By the term "CCR5 condition" is meant a disease state of the mammalian, especially human, body where CCR5 expression is upregulated or downregulated. Preferably, the CCR5 expression is upregulated since that should give better signal-to-background in diagnostic imaging *in vivo.* CCR5 expression is upregulated in chronic HIV infection. CCR5 conditions also include various pathological inflammatory conditions as well as neuroinflommatory conditions. Pathological inflammatory conditions include: atherosclerosis, chronic obstructive pulmonary disorder (COPD), rheumatoid arthritis, osteoarthritis, allergic disease, HIV/AIDS, asthma and cancer. Neuroinflammatory conditions include: multiple sclerosis (MS), Alzheimer's disease (AD) and Parkinson's disease (PD). A preferred method of the fifth aspect is the *in vivo* diagnosis or imaging of neuroinflammation. Most neurodegenerative diseases have an element of inflammation.

In a sixth aspect, the present invention provides the use of the pharmaceutical composition of the third aspect for imaging *in vivo* in a subject a CCR5 condition wherein said subject is previously administered with said pharmaceutical composition. The "CCR5 condition" and preferred embodiments thereof are as defined for the fifth aspect, above. By "previously administered" is meant that the step involving the clinician, wherein the imaging agent composition is given to the patient e.g. intravenous injection, has already been carried out.

In a seventh aspect, the present invention provides the use of the imaging agent of any one of the first aspect for the manufacture of a pharmaceutical for use in a method for the diagnosis of a CCR5 condition. The "CCR5 condition" and preferred embodiments thereof are as defined for the fifth aspect, above.

In an eighth aspect, the present invention provides a pharmaceutical composition for use in a method of monitoring the effect of treatment of a human or animal body with a drug to combat a CCR5 condition, said method comprising administering to said body the pharmaceutical composition of the third aspect, and detecting the uptake of the imaging agent of said pharmaceutical composition. The "CCR5 condition" and preferred embodiments thereof are as defined for the fifth aspect, above.

In a ninth aspect, the present invention provides the pharmaceutical composition of the invention for use in a method for the diagnosis of a CCR5 condition. The "CCR5 condition" and preferred embodiments thereof are as defined for the fifth aspect, above.

The invention is illustrated by the following Examples.

Example 1 provides the synthesis of a non-radioactive ¹⁹F counterpart reference compound ("Compound 1"). Since the ¹⁸F version differs only in the fluorine isotope, it is chemically almost identical.

Example 2 provides the synthesis of a non-radioactive ¹⁹F counterpart compound falling within Formula II of the present invention ("Compound 8").. Since the ¹⁸F version differs only in the fluorine isotope, it is chemically almost identical.

Examples 3 and 4 provide prophetic examples of the syntheses of ¹⁸F-labelled reference Compound 1 and Compound 8. Example 5 provides a prophetic example of the syntheses of an other reference ¹⁸F-labelled compound.

Example 6 provides biological screening data for Compound 8 of Example 2.. This shows that compound 8 binds CCR5 with high affinity and is selective for CCR5 as it does not bind CCR1 and CCR2B.

Example 7 provides the screening of reference Compound 1 and Compound 8 in a membrane permeability assay (PAMPA assay). Pe (permeability) predict a high CNS (blood brain barrier) permeability for Compound 8 and intermediate permeability for Compound 1.

### Abbreviations.

The following abbreviations are used:
DCM = dichloromethane.
DIAD = diisopropyl azodicarboxylate.
DIEA = diisopropylethylamine
DMF= N,N'-dimethylformamide.
EDCI = 1-(3-dimethylaminopropyl)-3-ethylcarbodiimide hydrochloride.
HOBT = 1-hydroxybenzotriazole.
LCMS = liquid chromatography mass spectroscopy.
THF = tetrahydrofuran.

### Examples.

### Reference Example 1: Synthesis of Compound 1.

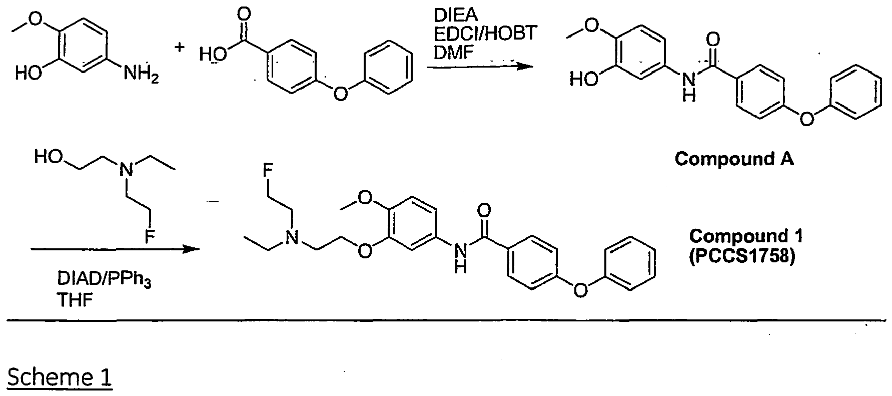

### Step (i): Synthesis of Compound A (Scheme 1).

A solution of 5-amino-2-methoxyphenol (2.78 g, 0.020 mol), 4-phenoxybenzoic acid (4.28 g, 0.020 mol), DIEA (3.1 g, 4.2 mL, 0.024 mol) and HOBT (3.2 g, 0.024 mol) in DMF (20 mL) was cooled to 0 °C, EDCI (4.6 g, 0.024 mol) was added in one portion under nitrogen. The mixture was stirred at room temperature overnight. The mixture was poured into ice-water (100 mL) and extracted with ethyl acetate (50 mL x 3). The combined ethyl acetate layer was washed with water, brine, dried (MgSO₄) and concentrated to dryness. The residue solid was triturated with DCM/hexanes, affording a white solid (4.1 g, 62%). ¹H NMR and LCMS analysis indicated >98% purity.

### Step (ii): Synthesis of 2-(ethyl-2'-fluoroethylamino)ethanol.

The required intermediate 2-(ethyl-2'-fluoroethylamino)ethanol was prepared as shown in Scheme 2.

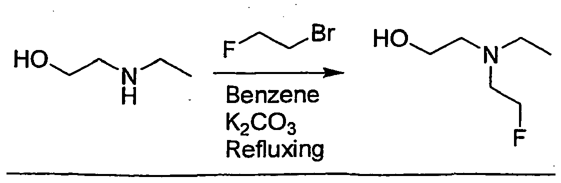

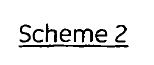

A mixture of 2-ethylaminoethanol (6.7 g, 0.075 mol), 2-fluoroethylbromide (12 g, 0.094 mol), anhydrous potassium carbonate (10.3 g, 0.075 mol) and dry benzene (50mL) was heated under reflux with stirring for 48 h. After cooling to room temperature, the solid was remove by filtration and washed with benzene. The benzene was removed *in vacuo*.¹H NMR analysis indicated the purity of the product was ~90% and it was used directly in the next step without any further purification. (9.0 g, 89%).

### Step (iii): Synthesis of Compound 1.

DIAD (1.36 g, 1.3 mL, 6.71 mmol) was added dropwise to a solution of Compound A from Step (i) (1.50 g, 4.47 mmol), 2-(ethyl-2'-fluoroethylamino)ethanol from Step (ii) (0.91 g, 6.71 mmol) and PPh₃ (1.76 g, 6.71 mmol) in anhydrous DCM (/THF (12 mL, 5:1). An exothermic reaction was immediately observed. The mixture was then stirred at room temperature overnight. The reaction mixture was concentrated to dryness and the residue solid was purified by column chromatography [silica, DCM →DCM/MeOH (98:2)]. The second fraction was the desired product, this fraction was chromatographed again under the same condition stated above. Recrystallisation from DCM/ hexanes afforded a colourless solid (0.81 g, 40%). Analytical data indicated >98% purity. LCMS (API-ES+) m/z 453.3 (M+H⁺)
Supporting Data: ¹H NMR, ¹³C NMR, HPLC, LCMS of Compound 1.

### Example 2: Synthesis of Compound 8.

Compound 8 was prepared according to Schemes 3 and 4:

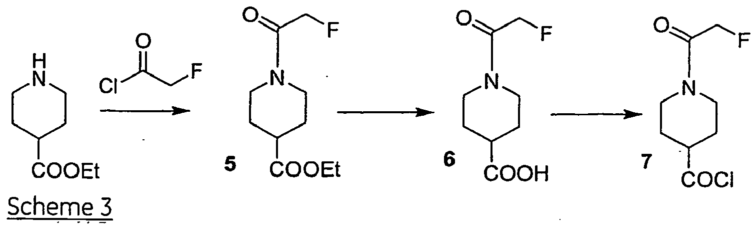

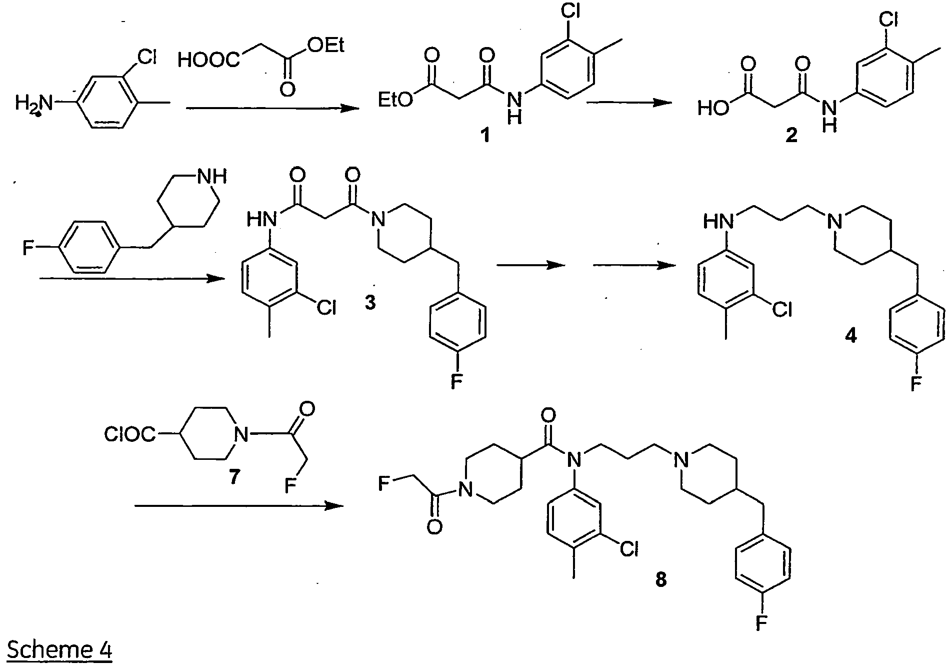

### Step (i): Synthesis of Compound 5:

Ethylpiperidine-4-carboxylate (10g, 0.064mol, 1.1eq) and triethylamine (15ml, 2eq) were dissolved in DCM (30ml), and cooled on ice water bath with magnetic stirring. 2-Fluoroacetylchloride (5g, 0.052mol, 1.0eq) in DCM (20ml) was added dropwise (15min) to the reaction and stirred for 1h. Water (100ml) was added and solvent was removed *in vacuo.* The residue was extracted into ethyl acetate (300ml), which was washed with water, 2N HCl, saturated NaHCO₃, brine and dried over Mg₂SO₄. The organic solution was filtered, then concentrated. After silica gel column chromatography 6.2g (yield 55%) of product compound 5 was obtained.

### Step (ii): Synthesis of Compound 6.

Compound **5** (6.2g, 0.029mol, 1eq) was dissolved in methanol (100ml) and 2N NaOH (30ml, 2eq) was added and stirred overnight. The methanol was then removed *in vacuo.* The residue was acidified with 2N HCl to pH 3, extracted with ethyl acetate (3 X 500ml), dried over MgSO₄. filtered and concentrated to afford compound **6** (4.3g, yield 78%).

### Step (iii): Synthesis of Compound 7.

Compound **6** (4.3g, 0.023mol) dissolved in DCM (50ml), and DMF (one drop) was added, cooled on ice water bath, followed by addition of oxalyl dichloride 12.3g, 1.5ml, 2.5eq) and stirred for 2h. Solvent was removed and dry toluene (50ml) was added to chase out possible residual solvent on a 50°C water bath to give compound **7** (4.2g, yield 88%).

### Step (iv): Synthesis of Compound 1 of Scheme 4.

3-chloro-4-methylbenzenamine (15g, 0.106mol, 1eq), 2-(ethoxycorbonyl)-acetic acid (14g, 0.106mol, 1eq), diisopropylethylamine (16.5g, 22.3ml, 1.2eq), HOBT(17g, 1.2eq) and DCM (150ml) were mixed together. [1-ethyl-(3-dimethyl-aminopropyl)carbodiimide hydrochloride anhydrous] (EDCl, 24.5g, 1.2eq) was then added, and the resulting mixture stirred overnight under N₂. Workup, the reaction mixture washed with water, saturated NaHCO_{3,} 1N HCl and brine, dried over MgSO₄. Filtered off and concentrated to afford compound **1** (18.5g, yield 70%).

### Step (v): Synthesis of Compound 2 of Scheme 4.

Compound **1** from step (iv) above (18.5g, ~0.072mol) was dissolved in methanol (100ml), and cooled on an ice water both. 2N NaOH (72ml, 2eq) was added and the mixture stirred overnight. Then solvent methanol was *removed in vacuo.* The residue was acidified with 2N HCl to pH 2 and extracted with ethyl acetate (500ml), dried over MgSO₄, filtered off and concentrated to give compound **2** (13g, 80%).

### Step (vi): Synthesis of Compound 3 of Scheme 4.

Compound **2** from step (v) (4.9g, ~0.021mol), 4-fluorobenzylpiperidine hydrochloride (4.9g, 0.021mol), diisopropylethylamine (12g, 2.2eq), HOBT (3.5g, 1.2eq) and DMF (150ml) were mixed together. [1-ethyl-(3-dimethyl-aminopropyl)carbodiimide hydrochloride anhydrous] (EDCl, 5g, 1.2eq) was then added. The resulting mixture was stirred overnight under N₂. Workup, the reaction mixture was diluted with water (800ml), extracted with ethyl acetate (500ml), washed with water, saturated NaHCO₃, 1N HCl and brine, dried over MgSO4. Filtered off and concentrated to afford compound **3** (6.8g, yield 80%).

### Step (vi): Synthesis of Compound 4 of Scheme 4.

Compound **3** from step (v) (6.8g, 0.017mol) was dissolved in THF (100ml), and BH₃ (1M solution in THF, 170ml, 10eq) was added and refluxed for 4 days till reaction complete (checked with LCMS). Solvent THF was then removed and methanol (100ml) 6N HCl (100ml) was added and refluxed for 5 days till reaction complete (checked with LCMS). Then methanol was removed and the residue was acidified to pH 11. Extracted with DCM (500ml), dried over MgSO4, filtered off and concentrated (crude 6.2g). After silica gel column give Compound **4** (3.7g, 58%).

### Step (vii): Synthesis of Compounds 8 of Scheme 4.

Compound **4** from step (vi) (2.5g, 0.0067mol) and triethylamine(7g, 10ml, 0.068mol, 10eq) was dissolved in DCM (50ml), cooled on ice water bath, added Compound 7 (4.2g, ~3eq) in DCM (50ml). The resulting mixture was stirred overnight Reaction is messy but LCMS showed the desired product molecular weight. Workup, water was added, solvent DCM was removed *in vacuo.* Residue was extracted with ethyl acetate (500ml), washed with water, saturated NaHCO₃, dried over MgSO₄, filtered off and concentrated, after silica gel column chromatography give final compound **8** (179mg, 5%). LCMS (API-ES+) m/z 546.3 (M+H+).

### Reference Example 3: Synthesis of ¹⁸F-labelled reference Compound 1 (prophetic example).

¹⁸F-labelled Compound 1 is prepared as shown in Scheme 5:

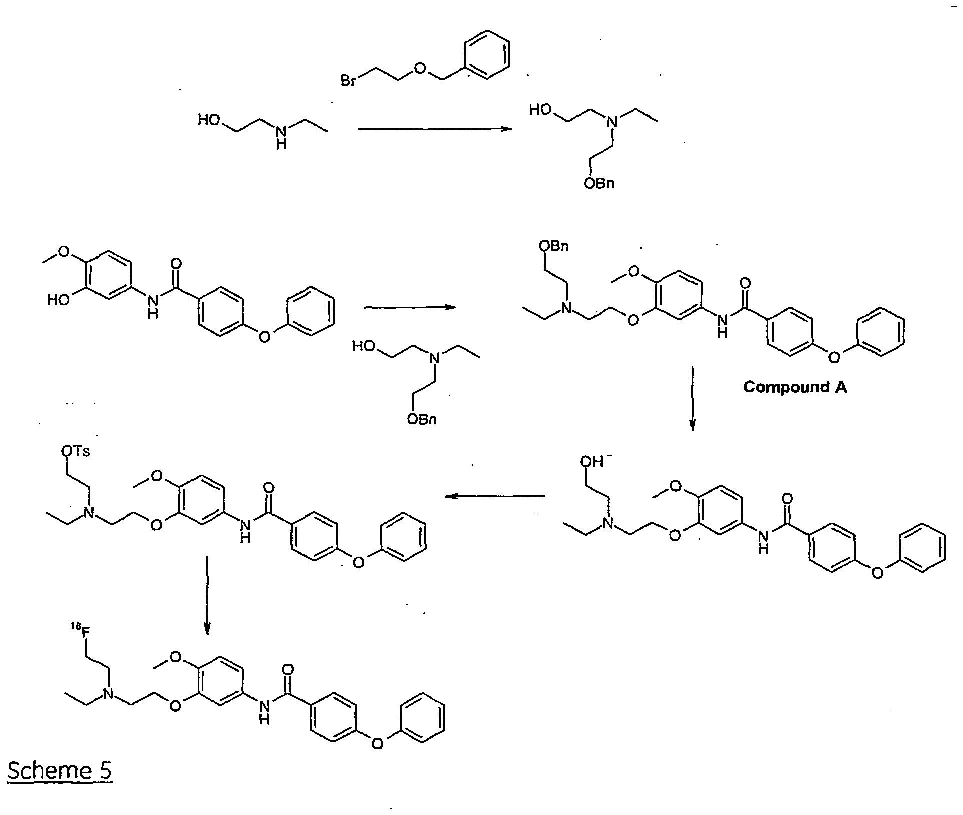

### Example 4: Synthesis of ¹⁸F-labelled Compound 8 (prophetic example).

The F-18 analogue of Compound 8 is synthesized from Compound 4 of Example 2 as shown in Scheme 6:

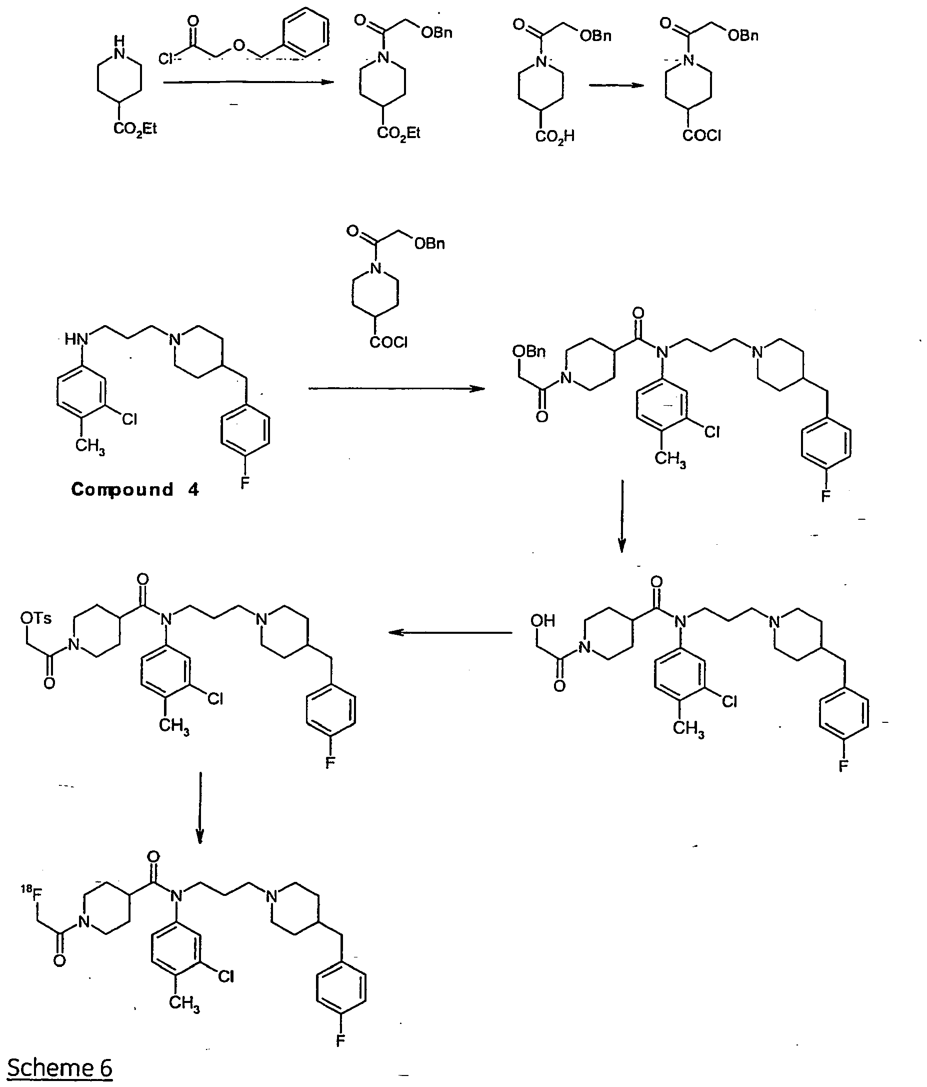

### Reference Example 5: Synthesis of ¹⁸F-labelled reference Compound (prophetic example).

An ¹⁸F-labelled reference Compound is prepared as shown in Scheme 7:

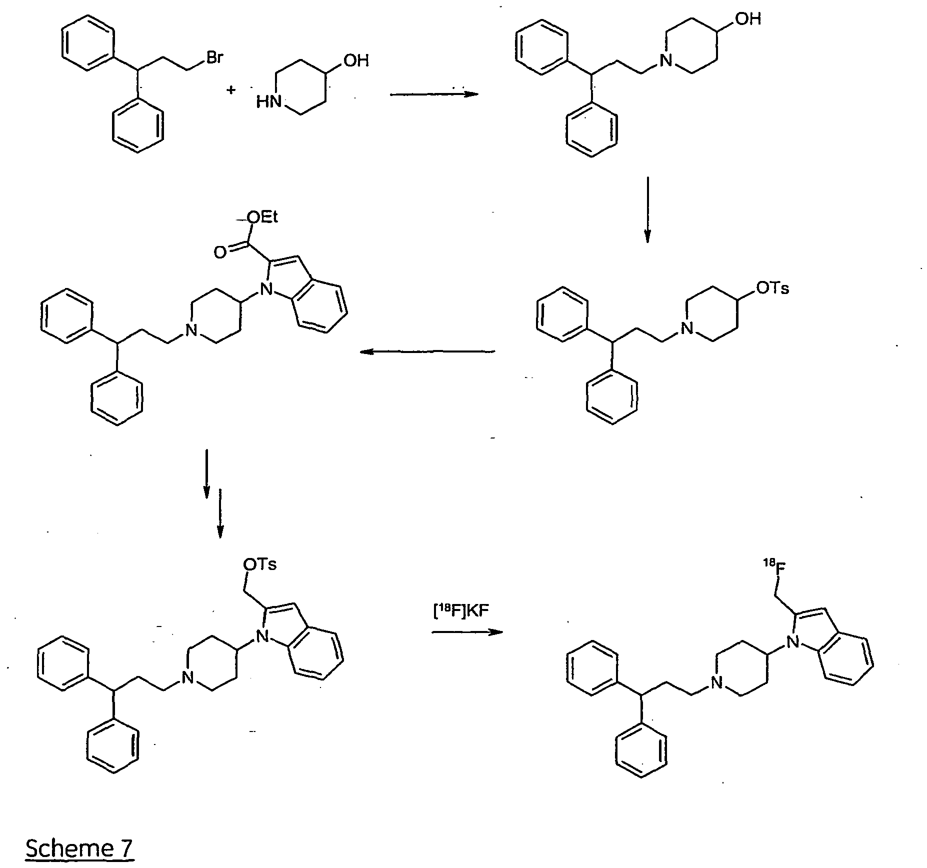

### Example 6: Screening of Compound 8.

Compound 8 of Example 2 was screened in CCR binding assays as follows:

The CCR1 binding assay was performed under the following conditions, according to a method that was adapted from the literature [Ben-Baruch et al., J. Biol. Chem., 270(38), 22123-8 (1995); Pease et al., J. Biol. Chem., 273(32), 19972-6 (1998)].

Thus, Compound 8 was incubated for 3 hours at 25°C in 50mM HEPES, pH7.4 containing 1mM CaCl₂, 0.5% BSA, 5mM MgCl₂ and 1% DMSO with Human recombinant CHO-K1 cells in the presence of 0.02nM [¹²⁵I]-MIP-1α. MIP-1α is Macrophage Inflammatory Protein 1α (ligand of CCR1 and CCR5).

CCR2B binding assay was performed under the following conditions, according to a method that was adapted from the literature [Gong et al., J. Biol. Chem., 272, 11682-5 (1997); Moore et al., J. Leukoc. Biol., 62, 911-5 (1997)].

Thus, Compound 8 was incubated for 1 hour at 25°C in 25mM HEPES, pH7.4 containing 1mM CaCl₂, 0.5% BSA, 5mM MgCl₂, 0.1% NaN₃ and 1% DMSO with Human recombinant CHO-K1 cells in the presence of 0.1nM [¹²⁵I]-MCP-1. MCP-1 is monocyte chemoattractant protein (the ligand of CCR2).

CCR5 binding assay was performed under the following conditions, according to a method that was adapted from the literature [Samson et al., J. Biol. Chem., 272, 24934-41 (1997)].

Thus, Compound 8 was incubated for 2 hours at 25°C in 50mM HEPES, pH7.4 containing 1mM CaCl₂, 0.5% BSA, 5mM MgCl₂ and 1% DMSOwith Human recombinant CHO-K1 cells in the presence of 0.1nM [¹²⁵I]-MIP-1β, where MIP-1β is Macrophage Inflammatory Protein 1β.

Compound 8 was found to be selective for CCR5 (Ki 0.79nM) since binding affinity for CCR1 was much lower (32% binding inhibition at 10µM Compound 8) and Compound 8 at 10µM concentration did not inhibit the binding of MCP-1 to CCR2B.

### Example 7: Permeability of Compound 8.

The permeability of the CCR compounds was measured in a Parallel Artificial Membrane Permeability Assay (PAMPA) which gives a prediction of the blood brain barrier penetration by passive diffusion [Di et al, Eur.J.Med.Chem., 38(3), 223-232 (2003)].

The commonly accepted classification ranges for this PAMPA assay are as follows:
- High predicted passive BBB permeation: Pe > 4.0 x 10⁻⁰⁶ cm/sec.
- Low predicted passive BBB permeation: Pe < 2.0 x 10⁻⁰⁶ cm/sec.

Uncertain prediction of BBB permeation: 2.0 x 10⁻⁰⁶ cm/sec < Pe < 4.0 x 10⁻⁰⁶ cm/sec.

The results were Pe = 3.2E-06 cm/sec for reference Compound 1 and Pe = 6.8E-06 cm/sec for Compound 8.

## Claims

1. An imaging agent which comprises a synthetic compound of Formula II: 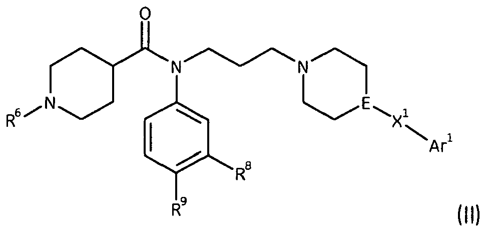 wherein:
R⁶ is [¹⁸F]fluoroacyl;
R⁸-R⁹ are independently selected from H, C₁₋₃alkyl, OH or halogen;
E is N or CH;
when E is N, X¹ is -CH₂- and when E is CH, X¹ is -CH₂- or -O-;
Ar¹ is a phenyl ring substituted with 0 to 3 R⁷ groups;
each R⁷ is independently chosen from C₁₋₃alkyl, OH, halogen, NO₂, NH₂, CO₂H, C₁-₆ alkoxy, C₁₋₆ amino, C₁₋₆ amido, -O(CH₂CH₂O)ₓX² or -NH(CH₂CH₂O)ₓX² where x is an integer of value 0 to 4, and X² is H or CH₃;
wherein said compound has affinity for chemokine receptor 5 (CCR5) and a molecular weight of 3000 Daltons or less.

2. A method for the preparation of the imaging agent of Claim 1, which comprises reaction of:
(i) a non-radioactive precursor; and,
(ii) a suitable source of ¹⁸F,
wherein said precursor is a derivative of the synthetic compound of Claim 1, and said derivative comprises a substituent Y¹ which is capable of reaction with said suitable source of ¹⁸F to give the imaging agent of claim 1.

3. A pharmaceutical composition which comprises the imaging agent of Claim 1 together with a biocompatible carrier, in a form suitable for mammalian administration.

4. The pharmaceutical composition of Claim 3 for use in a method for the *in vivo* diagnosis or imaging in a subject of a CCR5 condition, wherein said method comprises administration of said pharmaceutical composition.

5. The pharmaceutical composition of Claim 3 for use in a method of monitoring the effect of treatment of a human or animal body with a drug to combat a CCR5 condition, wherein said method comprises administering to said body the pharmaceutical composition of Claim 3 and detecting the uptake of the imaging agent of said pharmaceutical composition.

6. The pharmaceutical composition of Claim 3 for use in a method for the diagnosis of a CCR5 condition.

## Patentansprüche

1. Bildgebungsmittel, das eine synthetische Verbindung der Formel II umfasst: 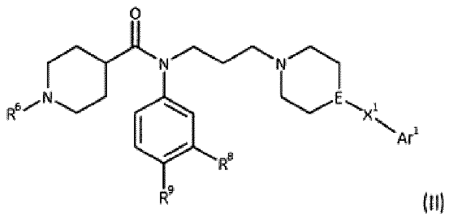 worin:
R⁶ für [¹⁸F]Fluoracyl steht;
R⁸-R⁹ unabhängig gewählt sind aus H, C₁₋₃-Alkyl, OH oder Halogen;
E für N oder CH steht;
wenn E für N steht, X¹ für -CH₂- steht, und wenn E für CH steht, X¹ für -CH₂-oder -O- steht;
Ar¹ für einen mit 0 bis 3 R⁷-Gruppen substituierten Phenylring steht;
jedes R⁷ unabhängig gewählt ist aus C₁₋₃-Alkyl, OH, Halogen, NO₂, NH₂, CO₂H, C₁₋₆-Alkoxy, C₁₋₆-Amino, C₁₋₆-Amido, -O(CH₂CH₂O)ₓX² oder - NH(CH₂CH₂O)ₓX², wo x für eine ganze Zahl mit Wert 0 bis 4 steht, und X² für H oder CH₃ steht;
wobei die Verbindung Affinität für Chemokinrezeptor 5 (CCR5) und eine Masse von 3000 Dalton oder weniger aufweist.

2. Verfahren zur Herstellung des Bildgebungsmittels nach Anspruch 1, das eine Umsetzung umfasst von:
(i) einem nicht radioaktiven Präkursor; und
(ii) einer geeigneten ¹⁸F-Quelle,
wobei der Präkursor ein Derivat der synthetischen Verbindung nach Anspruch 1 ist, und das Derivat einen Substituenten Y¹ umfasst, der zur Umsetzung mit der geeigneten ¹⁸F-Quelle befähigt ist, damit sich das Bildgebungsmittel nach Anspruch 1 ergibt.

3. Pharmazeutische Zusammensetzung, die das Bildgebungsmittel nach Anspruch 1 zusammen mit einem biokompatiblen Träger umfasst, in zur Verabreichung an Säuger geeigneter Form.

4. Pharmazeutische Zusammensetzung nach Anspruch 3 zur Verwendung in einem Verfahren für die *in vivo* Diagnose oder Bildgebung einer CCR5-Störung bei einem Versuchslebewesen bzw. Patienten, wobei das Verfahren die Verabreichung der pharmazeutischen Zusammensetzung umfasst.

5. Pharmazeutische Zusammensetzung nach Anspruch 3 zur Verwendung in einem Verfahren zur Überwachung der Wirkung einer Behandlung eines menschlichen oder tierischen Körpers mit einem Arzneimittel, um eine CCR5-Störung zu bekämpfen, wobei das Verfahren das Verabreichen der pharmazeutischen Zusammensetzung nach Anspruch 3 an den Körper und das Erfassen der Aufnahme des Bildgebungsmittels der pharmazeutischen Zusammensetzung umfasst.

6. Pharmazeutische Zusammensetzung nach Anspruch 3 zur Verwendung in einem Verfahren für die Diagnose einer CCR5-Störung.

## Revendications

1. Agent d'imagerie qui comprend un composé synthétique de formule II 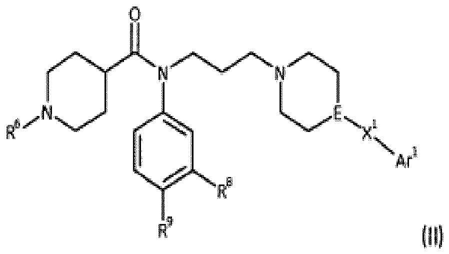 dans laquelle :
R⁶ est représente du [¹⁸F] fluoroacyle ;
R⁸ et R⁹ sont sélectionnés indépendamment à partir de H, un alkyle en C₁₋₃, OH ou un halogène ;
E représente N ou CH ;
lorsque E représente N, X¹ représente -CH₂- et lorsque E représente CH, X¹ représente -CH2- ou -O- ;
Ar¹ est un cycle phényle substitué avec 0 à 3 groupes R⁷;
chaque groupe R⁷ est sélectionné indépendamment à partir d'un alkyle en C₁₋₃, de OH, d'un halogène, de NO₂, NH₂, CO₂H d'un alkoxy en C₁₋₆, d'un amino en C₁₋₆, d'un amido en C₁₋₆, de -O(CH₂CH₂O)ₓX₂ ou de - NH(CH2CH2O)xX2 où x représente un entier d'une valeur de 0 à 4, et R² représente H ou CH₃ ;
dans lequel ledit composé présente une affinité pour le récepteur 5 de la chémokine (CCR5) et un poids moléculaire de 3000 Daltons ou inférieur.

2. Procédé de préparation de l'agent d'imagerie selon la revendication 1, qui comprend la réaction de :
(i) un précurseur non radioactif ; et,
(ii) une source appropriée de ¹⁸F,
dans lequel ledit précurseur est un dérivé du composé synthétique de la revendication 1, et ledit dérivé comprend un substituant Y¹ qui est capable de réagir avec ladite source appropriée de ¹⁸F afin d'obtenir l'agent d'imagerie de la revendication 1.

3. Composition pharmaceutique qui comprend l'agent d'imagerie selon la revendication 1, ensemble avec un support biocompatible, sous une forme appropriée à l'administration mammifère.

4. Composition pharmaceutique selon la revendication 3 destinée à une utilisation dans un procédé de diagnostic ou d'imagerie in vivo d'un sujet dans une condition CCR5, dans laquelle ledit procédé comprend l'administration de ladite composition pharmaceutique.

5. Composition pharmaceutique selon la revendication 3 destinée à une utilisation dans un procédé de surveillance de l'effet d'un traitement d'un corps humain ou animal avec un médicament afin de combattre une condition CCR5, dans laquelle ledit procédé comprend l'administration sur ledit corps de la composition radio-pharmaceutique de la revendication 3 et la détection de l'assimilation de l'agent d'imagerie de ladite composition pharmaceutique.

6. Composition pharmaceutique selon la revendication 3 destinée à une utilisation dans un procédé de diagnostic d'une condition CCR5.
